# EUROPEAN PATENT APPLICATION

(11) **EP 4 410 229 A1**
(43) Date of publication of application: **07.08.2024**
(21) Application number: 24153499.9
(22) Date of filing: 23.01.2024
(51) Int. Cl.: A61B 18/02, A61B 18/14, A61B 18/18, A61B 90/13, A61B 90/00

(54) **ABLATION PROBES WITH GUIDANCE INDICATORS TO SUPPORT LOCATION AND DIRECTION GUIDANCE SYSTEMS**

(30) Priority: 02.02.2023 US 202318163531
(71) Applicant: Varian Medical Systems Inc, Palo Alto CA 94304 (US)
(72) Inventor: HATTAN, Paul, Minneapolis, 55401 (US); JESTER, Steven, Eden Prairie, 55346 (US)
(74) Representative: Bartholomew, Anna

(57) **Abstract**

A guidance indicator (3004, 3104, 3400) includes an indicator platform (3008, 3108, 3402) having an indicator surface and a mounting portion (3016) connected to the indicator platform and supporting the indicator surface in a predetermined orientation relative to a needle (3006, 3106, 3504) of an ablation probe (3002, 3102, 3502). The mounting portion is adapted to removably attach to a complimentary attachment post (3020, 3120, 3506) of the ablation probe.

## Description

### TECHNICAL FIELD

The disclosure relates to devices and features of ablation probes to support location and direction guidance systems.

### BACKGROUND

Ablation probes are instruments used in medical procedures. During some such medical procedures, the ablation probe can be used to scar, destroy or otherwise treat a target tissue such as a tumor or other abnormal tissue. Ablation procedures can include cryoablation, radio frequency (RF) ablation, microwave ablation and the like. During such procedures using these probes, the probes can be inserted into a subject body and positioned at a desired position relative to the target tissue. Various types of guidance systems can be used to assist medical professionals in the positioning of such probes relative to the target tissue. Examples of such systems can include imaging systems such as ultrasound, magnetic resonance imaging (MRI), computerized tomography (CT), and other systems. In addition, the guidance systems can include other indicators or other guidance tools to assist the medical professional in positioning the probe relative to the target tissue.

Existing ablation probes can be difficult to position relative to the target tissue so as to position the probe in a desired position and to minimize damage and/or proximity to surrounding healthy tissue or other body structures. There exists a need, therefore, for improved ablation probes with complimentary devices and features to allow medical professionals to more effectively position ablation probes in desired positions using guidance tools and imaging systems.

### SUMMARY

The methods and apparatuses described herein are directed to embodiments and examples that can be used to position an ablation probe in a preferred location and/or preferred orientation using a guidance system.

In accordance with some embodiments a guidance indicator is provided. The guidance indicator may include an indicator platform having an indicator surface and a mounting portion connected to the indicator platform and supporting the indicator surface in a predetermined orientation relative to a needle of an ablation probe. The mounting portion may be adapted to removably attach to a complimentary attachment post of the ablation probe.

In one aspect, the mounting portion may include a retention cup that includes a magnet to removably couple the guidance indicator to the attachment post.

In another aspect, the magnet may be configured to removably couple to a complimentary magnet in the attachment post.

In another aspect, the retention cup may define a cavity to receive the attachment post.

In another aspect, the mounting portion may be connected at a center of the indicator platform opposite to the indicator surface.

In another aspect, the indicator surface may include a first marking and a second marking. The second marking may be oriented perpendicular to the first marking. The first marking and the second marking may be configured to align to one or more lasers of a laser guidance system.

In another aspect, the predetermined orientation may be an orientation of the indicator surface that is substantially perpendicular to an axis of the needle.

In another aspect, the mounting portion may include a magnet positioned at a base of a retention cup to removably engage to the attachment post of the ablation probe.

In another aspect, the retention cup may include a wall, the wall and the base defining a cavity of the retention cup.

In another aspect, the mounting portion may be configured to removably attach to the attachment post via an interference fit.

In another aspect, the mounting portion may include one or more guide ribs positioned proximate a coupling ring, the guide ribs angled toward a center of the coupling ring to guide the attachment post into the coupling ring during assembly of the guidance indicator to the ablation probe.

In another aspect, the mounting portion may include a retention cup and a center of the retention cup is aligned with an axis of the needle when positioned on the ablation probe.

In another aspect, the guidance indicator may also include a handle connected to the indicator platform on a side opposite to the indicator surface.

In some embodiments, an ablation probe is provided and the ablation probe may include the guidance indicators of the present disclosure.

In one aspect, the attachment post may be made of an elastic material.

In another aspect, the attachment post may be flexible and biased to maintain the indicator surface in the predetermined orientation.

In another aspect, the attachment post may include a circular support configured to be removably engaged in the retention cup.

In another aspect, the ablation probe may include a handle. The needle may extend away from the handle on a first side and the attachment post may extend away from the handle on a second side opposite to the first side.

In another aspect, an overall width of the indicator surface may be greater than a width of the second side of the handle.

In another aspect, the overall width of the indicator surface may be at least twice the width of the second side of the handle.

In some embodiments in accordance with the present disclosure, an ablation probe may include an elongated needle portion comprising a distal end to be positioned at a target tissue and a proximal end positioned away from the distal end. The probe may also include a guidance indicator located at the proximal end that is aligned with the needle portion.

In one aspect, the guidance indicator may include a planar surface oriented substantially perpendicular to an axis of the needle portion.

In another aspect, the guidance indicator can be removed from the needle portion.

In another aspect, the guidance indicator may include a first marking and a second marking. The second marking is oriented perpendicular to the first marking. The first marking and the second marking are configured to align to one or more lasers of a laser guidance system.

In another aspect, the ablation probe may include a handle coupled to the needle portion at the proximal end, wherein the guidance indicator is positioned on the handle.

In some embodiments, the probe is a cryoablation probe.

In some embodiments, the probe is a radio frequency (RF) ablation probe.

In some embodiments, the probe is a microwave ablation probe.

In another aspect, the guidance indicator may include a target portion connected to an elongated bar and the elongated bar is configured to removably engage with a handle of the probe.

In another aspect, the guidance indicator may include a target portion and a support portion. The support portion may be made of an elastomeric material configured to removably engage the probe via an interference fit.

In another aspect, the guidance indicator may include at least one optical sensor and is configured to determine a predetermined alignment of the probe.

In another aspect, the guidance indicator may include an array of optical sensors and at least one alignment notification, the at least one alignment notification configured to indicate alignment of the probe to a reference of a guidance system.

In some embodiments in accordance with the present disclosure, a guidance indicator for use with an ablation probe is provided. The guidance indicator may include an attachment portion configured to removably attach to the ablation probe and at least one marking connected to the attachment portion. The attachment portion and the at least one marking are positioned such the at least one marking is positioned at a predetermined location relative to a needle of the ablation probe when the attachment portion is attached to the ablation probe.

In one aspect, the predetermined location corresponds to an alignment of the at least one marking with a central axis of the needle of the ablation probe.

In another aspect, the attachment portion may include an elastomeric sleeve configured to connect to the ablation probe via an interference fit.

In another aspect, the attachment portion may include an elongated bar and the at least one marking is positioned on a target portion connected to the elongated bar. The elongated bar may be configured to attach to a sleeve of the ablation probe.

In some embodiments of the present disclosure, a method of positioning an ablation probe is provided. The method may include aligning a distal end of the probe relative to a subject using a first reference from a guidance system and aligning a proximal end of the probe relative to the subject using a second reference from the guidance system and a guidance indicator on the probe.

In another aspect, the method may include attaching the guidance indicator to the proximal end of the probe.

In another aspect, the method may include aligning at least one marking on the guidance indicator with the second reference from the guidance system.

In another aspect, the step of aligning the proximal end of the probe may include determining a predetermined alignment using at least one optical sensor in the guidance indicator.

### BRIEF DESCRIPTION OF THE DRAWINGS

The features and advantages of the present disclosures will be more fully disclosed in, or rendered apparent by the following detailed descriptions of example embodiments. The detailed descriptions of the example embodiments are to be considered together with the accompanying drawings wherein like numbers refer to like parts and further wherein:
FIG. 1 is an illustration of a subject positioned at a treatment station that includes a probe guidance system.
FIG. 2 is an image showing an example first reference for the positioning of a distal end of an ablation probe produced by a probe guidance system.
FIG. 3 is an image showing an example second reference for the positioning of a proximal end of an ablation probe produced by the probe guidance system.
FIG. 4 is an image showing the second reference of FIG. 3 aligned with the proximal end of an ablation probe.
FIG. 5 is an image the first reference aligned with the distal end of the ablation probe and the second reference aligned with the proximal end of the ablation probe in a subject.
FIG. 6 is an image of an example ablation probe that includes a molded handle.
FIG. 7 is an image showing a magnified view of the proximal end of the ablation probe and handle of FIG. 6.
FIG. 8 is an image showing another example ablation probe.
FIG. 9 is an image showing a magnified view of the proximal end of the ablation probe of FIG. 8.
FIG. 10 is an illustration of an example ablation probe with a guidance indicator in accordance with the present disclosure.
FIG. 11 is a top view of the proximal end of the ablation probe of FIG. 10.
FIG. 12 is an illustration of another example ablation probe with a raised guidance indicator in accordance with the present disclosure.
FIG. 13 is a top view of the ablation probe of FIG. 12.
FIG. 14 is an illustration of another example ablation probe with a guidance indicator in accordance with the present disclosure.
FIG. 15 is another view of the ablation probe of FIG. 15.
FIG. 16 is a top view of the ablation probe of FIG. 14.
FIG. 17 is an image showing another example ablation probe with another example guidance indicator in accordance with the present disclosure.
FIG. 18 is an image showing the ablation probe and guidance indicator of FIG. 17 with the guidance indicator removed.
FIG. 19 is an image showing another example ablation probe with another example guidance indicator in accordance with the present disclosure.
FIG. 20 is an image showing the ablation probe and guidance indicator of FIG. 19 with the guidance indicator removed.
FIG. 21 is an illustration of another example ablation probe and guidance indicator in accordance with the present disclosure.
FIG. 22 is an illustration of another example ablation probe and guidance indicator in accordance with the present disclosure.
FIG. 23 is an illustration of another example ablation probe and guidance indicator in accordance with the present disclosure.
FIG. 24 is an illustration of another example ablation probe and guidance indicator in accordance with the present disclosure.
FIG. 25A is an illustration of an example guidance indicator in accordance with the present disclosure.
FIG. 25B is an illustration of the guidance indicator of FIG. 25A showing misalignment with a reference of a guidance system.
FIG. 25C is an illustration of the guidance indicator of FIG. 25A showing alignment with a reference of a guidance system.
FIG. 26 is an illustration of another example guidance indicator in accordance with the present disclosure.
FIG. 27 is an illustration of an example ablation probe with the guidance indicator of FIG. 26.
FIG. 28 is a plan view of the example ablation probe and guidance indicator of FIG. 27.
FIG. 29 is an illustration of the example ablation probe and guidance indicator of FIG. 27 shown being aligned with a guidance system.
FIG. 30 is an illustration of another example ablation probe and guidance indicator in accordance with the present disclosure.
FIG. 31 is an illustration of another example ablation probe and guidance indicator in accordance with the present disclosure.
FIG. 32 is an illustration of another view of the example ablation probe and guidance indicator of FIG. 31.
FIG. 33 is an illustration of the example guidance indicator of FIG. 31 disengaged from the ablation probe.
FIG. 34 is an illustration of another example guidance indicator in accordance with the present disclosure.
FIG. 35 is an illustration of the example guidance indicator of FIG. 34 installed on an ablation probe.
FIG. 36 is a side view of the guidance indicator of FIG. 34 installed on an ablation probe.
FIG. 37 is an illustration of another example guidance indicator in accordance with the present disclosure.
FIG. 38 is an illustration of another example guidance indicator in accordance with the present disclosure.

### DETAILED DESCRIPTION

The description of the preferred embodiments is intended to be read in connection with the accompanying drawings, which are to be considered part of the entire written description of these disclosures. While the present disclosure is susceptible to various modifications and alternative forms, specific embodiments are shown by way of example in the drawings and will be described in detail herein. The objectives and advantages of the claimed subject matter will become more apparent from the following detailed description of these exemplary embodiments in connection with the accompanying drawings.

It should be understood, however, that the present disclosure is not intended to be limited to the particular forms disclosed. Rather, the present disclosure covers all modifications, equivalents, and alternatives that fall within the spirit and scope of these exemplary embodiments. The terms "couple," "coupled," "operatively coupled," "operatively connected," and the like should be broadly understood to refer to connecting devices or components together either mechanically, electrically, wired, wirelessly, or otherwise, such that the connection allows the pertinent devices or components to operate (e.g., communicate) with each other as intended by virtue of that relationship.

In the present disclosure the singular forms "a," "an," and "the" include the plural reference, and reference to a particular numerical value includes at least that particular value, unless the context clearly indicates otherwise. When values are expressed as approximations, by use of the antecedent "about," it will be understood that the particular value forms another embodiment. As used herein, "about X" (where X is a numerical value) preferably refers to ±10% of the recited value, inclusive. For example, the phrase "about 8" preferably refers to a value of 7.2 to 8.8, inclusive. Where present, all ranges are inclusive and combinable. For example, when a range of "1 to 5" is recited, the recited range should be construed as including ranges "1 to 4", "1 to 3", "1-2", "1-2 & 4-5", "1-3 & 5", "2-5", and the like. In addition, when a list of alternatives is positively provided, such listing can be interpreted to mean that any of the alternatives may be excluded, e.g., by a negative limitation in the claims. For example, when a range of "1 to 5" is recited, the recited range may be construed as including situations whereby any of 1, 2, 3, 4, or 5 are negatively excluded; thus, a recitation of "1 to 5" may be construed as "1 and 3-5, but not 2", or simply "wherein 2 is not included." It is intended that any component, element, attribute, or step that is positively recited herein may be explicitly excluded in the claims, whether such components, elements, attributes, or steps are listed as alternatives or whether they are recited in isolation.

In accordance with various embodiments of the present disclosure, an ablation probe is provided that can include a guidance indicator. The guidance indicator can allow a medical professional to align the ablation probe in a predetermined orientation and/or location on a subject to assist the medical professional in guiding the distal end of the probe to a desired location in the subject. Typically, the desired location is at or near a target or abnormal tissue such as a tumor. The guidance indicator that may be included on or can be used in conjunction with the ablation probe and can make the alignment and positioning of the ablation probe more easy and more accurate than existing methods and existing probes and related devices.

The ablation probes and/or the guidance indicators of the present disclosure can be used during treatments in which a guidance system is used to align and position the ablation probe. In some example guidance systems, one or more guiding references can be projected or displayed, such as by a laser, on the subject. The medical professional can use the guiding references to position the probe on the subject and then to insert the probe at a desired orientation relative to the subject. The use of such guidance systems can improve the ability of the medical professional to insert the needle of the ablation probe into the subject to position the distal end of the ablation probe at the target tissue. The guidance system can also guide the medical professional to avoid unnecessary or undesirable damage to healthy tissue while the probe is being inserted into the subject.

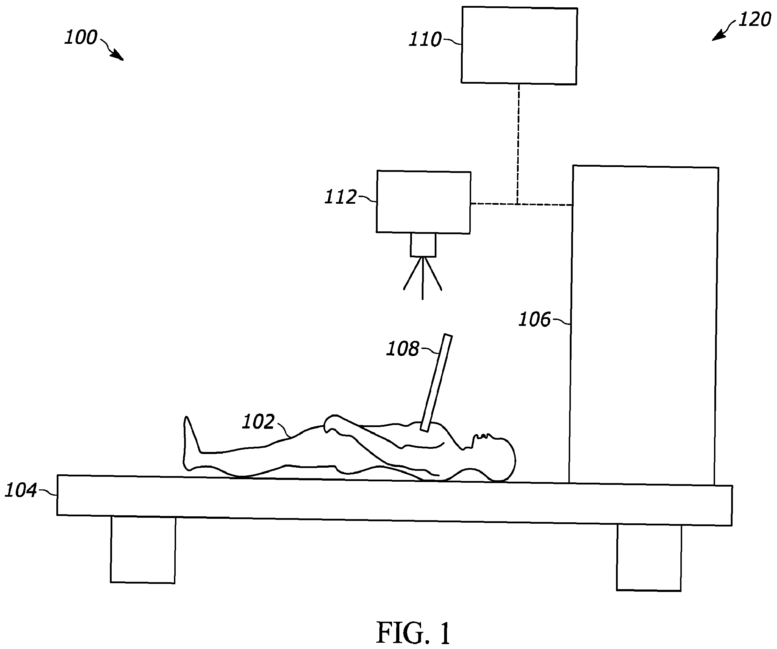

Referring now to FIG. 1, an example treatment station 100 is shown. A subject 102 can be positioned on a treatment bed 104 during an ablation procedure. The treatment station 100 can include an imaging system 106. The imaging system 106 can be any suitable system or device that can be used to capture images of the subject that can be used to determine a desired location and/or insertion path for the ablation probe 108. The imaging system can be, for example, a computerized tomography (CT) scanner, magnetic resonance imaging (MRI) scanner, x-ray scanner, an ultrasound imaging device or other imaging device. For various types of ablation procedures, different imaging devices may be used.

As further shown, the imaging system 106 can be coupled to a guidance system 120 that can include a computing device 110 and a guidance projector 112. The computing device 110 can be any suitable computer, server, or other device including a processor and memory that can receive data from the imaging device 106 and determine a path and/or positioning for the ablation probe 108 in the subject 102 for a desired treatment. The computing device 110 can be coupled to the guidance projector 112 that can project one or more references onto the subject 102 to assist the medical professional that is performing the treatment procedure. In one example, the guidance projector 112 can include one or more lasers that can project a reference such as a line, target, crosshair or other suitable marking onto the subject. The medical professional can then use the one or more references displayed by the guidance projector 112 to position the ablation probe 108 and insert the ablation probe 108 into the subject 102 to perform the treatment. One example of a guidance system 120 is a laser guidance system provided by Siemens that utilizes a software known under the name my-needle^{™}. The probes and guidance indicators of the present disclosure can be used in conjunction with this system and software as well as other guidance systems.

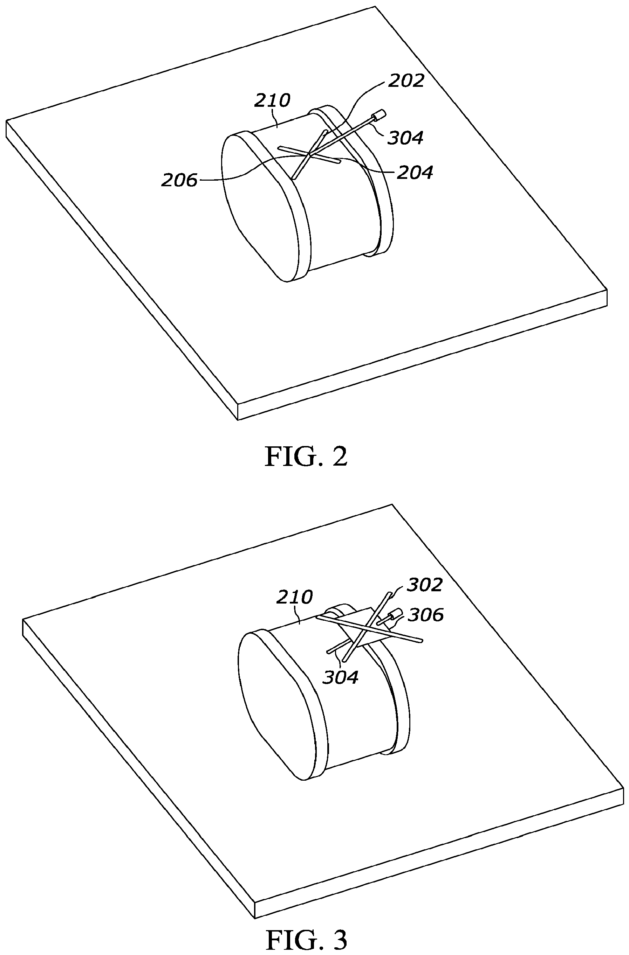

Referring now to FIGs. 2-5, images of an example guidance system are shown. As shown in FIG. 2, the guidance system can display a first reference 206 onto a subject 210. The subject 210 may be a patient or body structure, for example. In the image shown, the subject 210 is shown as a body analogue for ease of description and illustration purposes only. The first reference 206, in this example, includes a first line 202 and a second line 204 that intersect to form the first reference 206 in the shape of a crosshair. In other examples guidance systems, the first reference 206 can include other markings, targets, symbols or the like. The first reference 206 indicates to the medical professional where the distal end of the ablation probe should be placed for an entrance point on the subject 210.

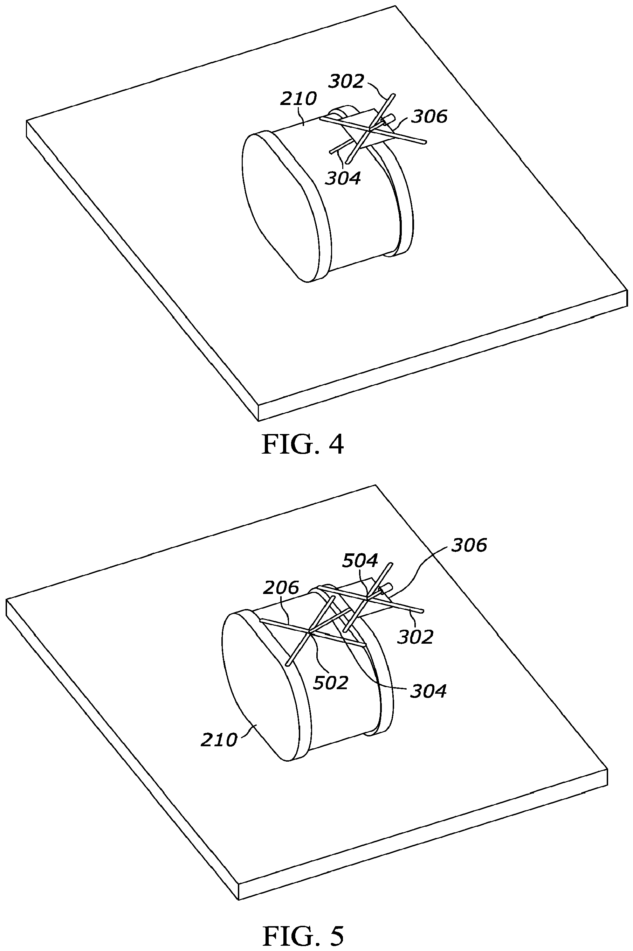

FIG. 3 shows a second reference 302 that can be displayed by the guidance system. The second reference 302 can be configured similarly to the first reference 206 previously described. The second reference 302 can indicate to the medical professional where a proximal end of the ablation probe 304 should be aligned to position the ablation probe at a desired trajectory for insertion of the probe. In still other examples, a single laser can project a single reference that can be used as the first reference 206 and the second reference. In other examples, multiple lasers can be used in the guidance system. In the example shown, the probe 304 lacks suitable surface area for alignment so the operator uses a piece of paper or other supplemental surface 306 to align the probe 304 with the second reference 302. FIG. 4 shows the proximal end of the ablation probe 304 aligned with the second reference 302.

Referring now to FIG. 5, the probe 304 has been aligned in a desired position and orientation relative to the subject 210. In the aligned position, the distal end 502 of the probe 304 is positioned and aligned with the first reference 206 and the proximal end 504 of the probe 304 is aligned with the second reference 302.

In addition to an entrance point and or starting position, the guidance system can display additional references and/or provide feedback to a user that the probe 304 is in an intermediate position and/or a final position. The guidance system can use an iterative process to image the subject and/or a position of the probe 304. In this manner, the guidance system can provide additional information to a medical professional regarding a position of the probe relative to the target tissue and/or relative to adjacent tissues and body structures during execution of an ablation procedure.

Many existing ablation probes suffer from drawbacks when used in connection with the guidance systems described above and shown in FIGs. 2-5. Existing ablation probes lack suitable surfaces and/or complimentary features to allow a medical professional to utilize the advanced features and reference displays of guidance systems. For example, many existing ablation probes are not suited to allow a medical professional to easily align the ablation probes with a first reference or a second reference such as the laser crosshair references previously described.

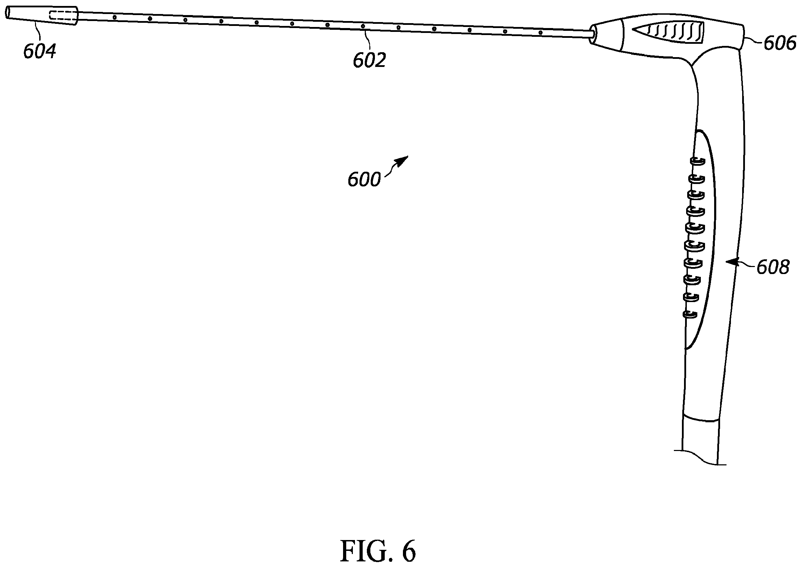

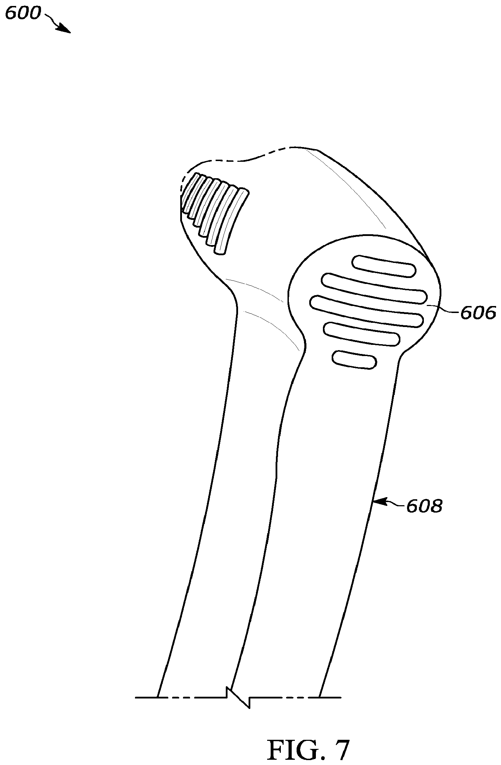

Examples of existing ablation probes are shown in FIGs. 6-9. In some existing examples, as shown in FIGs. 6 and 7, an ablation probe 600 can include a needle 602 with a distal end 604, a proximal end 606 and a molded handle 608. While the proximal end 606 of the probe 600 includes a flattened surface it does not include a center marking or other alignment feature to assist a medical professional in aligning the probe 600 using a guidance system.

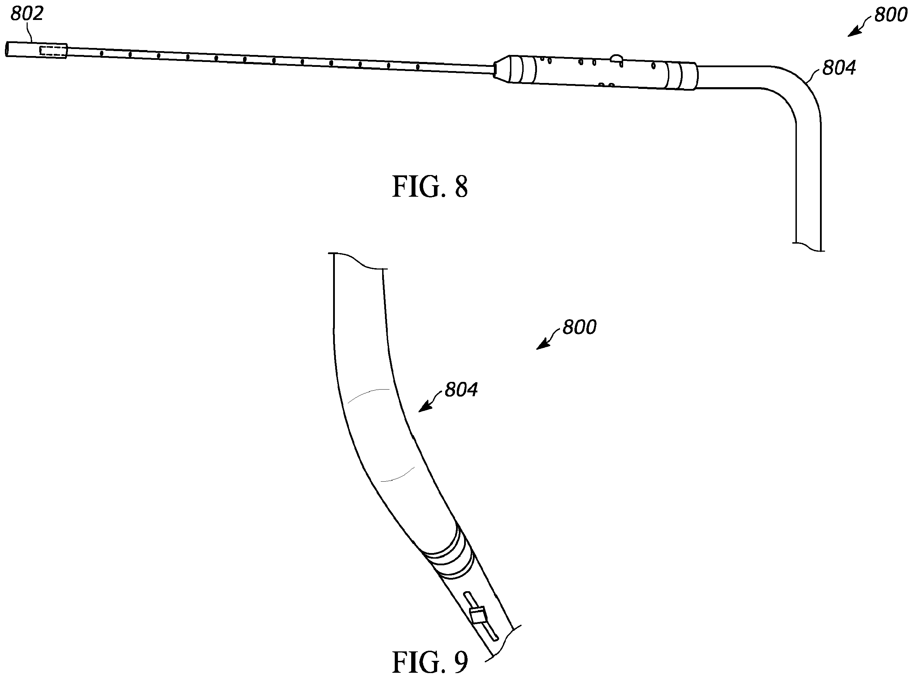

As shown in the example of FIGs. 8-9, an ablation probe 800 can include a distal end 802 and a proximal end 804. In this example, the proximal end 804 of the probe can be formed of bent tubing that may include a cover material or other insulation but does not include a marking or other alignment feature to assist a medical professional in aligning the probe 800 using a guidance system.

The ablation probes of the present disclosure can include a guidance indicator that can be used to align the probe using a guidance system such as the guidance systems previously described. In some examples, the guidance indicator can be formed or marked on the ablation probe. In other examples, the guidance indicator can be attached and removed from the ablation probe as desired during performance of the treatment procedure. The ablation probes and the guidance indicators of the present disclosure are improvements over existing probes and methods of use because the ablation probes and guidance indicators of the present disclosure can be used in conjunction with guidance systems to accurately and reliably position the probe relative to the subject at a desired location and at a desired orientation. Such accurate and reliable positioning can result in improved treatment results such as a greater likelihood of destruction of the target tissue and in a reduced likelihood of damage to surrounding tissue and surrounding body structures.

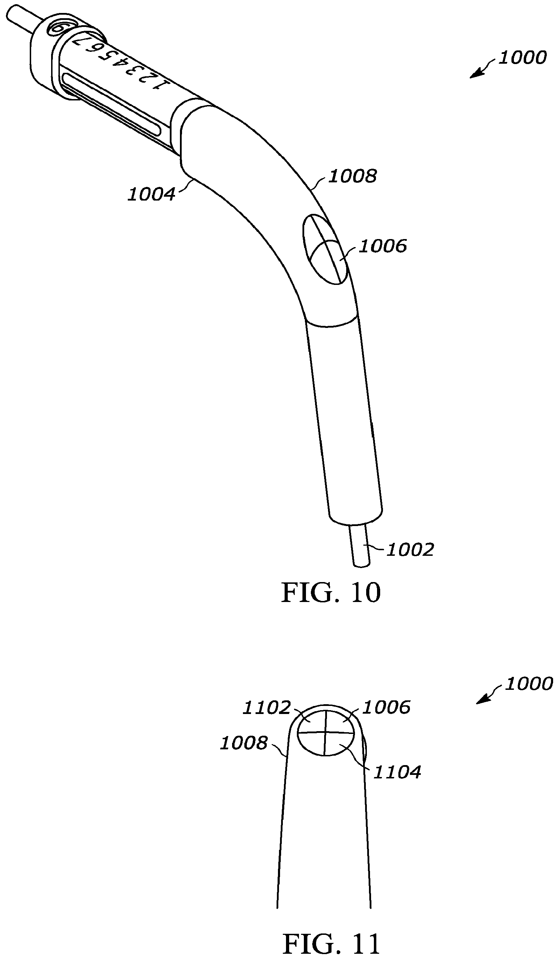

Referring now to FIGs. 10 and 11 an example ablation probe 1000 is shown. The ablation probe 1000 can include a needle 1002 and a handle portion 1004. The needle 1002 can include a distal end (not shown) that can extend away from a proximal end 1008. As further shown, the ablation probe 1000 can include a guidance indicator 1006 that is positioned at the proximal end 1008. The guidance indicator 1006 can be used to align the probe 1000 with or using a reference (e.g., the second reference 302, see FIG. 3) that may be projected or displayed by a guidance system. The guidance indicator 1006 can be printed or otherwise fixed on the handle portion 1004. The guidance indicator 1006 can include a target symbol or other marking that can be aligned with a crosshair indicator. In other examples, the guidance indicator 1006 can have other markings, targets or symbols that can be used to align the probe 1000 with displayed reference of a guidance system.

As further shown in this example, the guidance indicator 1006 can be printed or otherwise configured so that when the guidance indicator 1006 is viewed from a top or plan view (see FIG. 11), the guidance indicator 1006 can appear as one or more perpendicularly oriented markings. For example, the guidance indicator 1006 can be elongated or appear distorted when viewed from the side or from other oblique angle as shown in FIG. 10. When viewed from above, however, the guidance indicator 1006 can show a first marking 1102 and a second marking 1104 that appear as perpendicularly oriented markings as shown in FIG. 11. In other examples, the guidance indicator 1006 can be configured to have other markings that can facilitate use with other reference displays of other guidance systems.

In this example, the guidance indicator 1006 is positioned on a curved or non-planar surface of the ablation probe 1000. The guidance indicator 1006 can be positioned on a surface that curves away from the linear needle 1002 of the ablation probe 1000. In other examples, the guidance indicator 1006 can be positioned on other surfaces or at other locations on the ablation probe 1000. The guidance indicator 1006 can be printed on the handle portion 1004 using a suitable ink or other contrasting material. The guidance indicator can include an ink or other material that can glow or otherwise indicate alignment with a laser reference or other reference a guidance system, such as an iridium ink or material. In other examples, the guidance indicator 1006 can be fixed using adhesive, as a sticker or other suitable attachment. In still other examples, the guidance indicator 1006 can be stamped or integrally formed into the handle portion 1004. In yet other examples, the guidance indicator 1006 can be laser marked and/or embossed or debossed on the surface of the probe 1000.

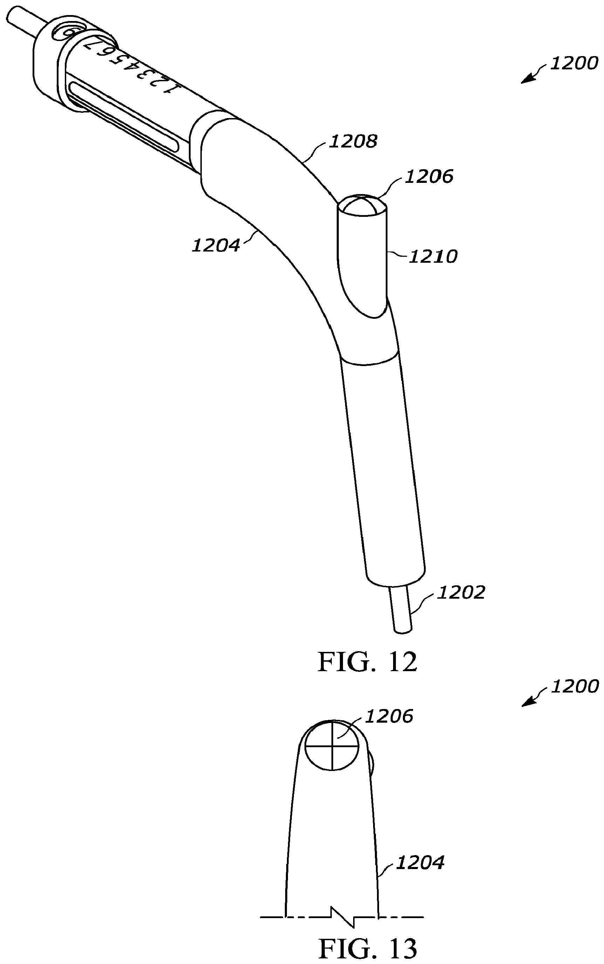

Another example ablation probe 1200 is shown in FIGs. 12 and 13. In this example, the ablation probe 1200 includes a needle 1202 and a handle portion 1204. The ablation probe 1200 can be similar to the ablation probe 1000 previously described. The ablation probe 1200 can include a guidance indicator 1206 positioned at or near a proximal end 1208 of the probe 1200. The guidance indicator 1206, in this example, is positioned on an indicator mount 1210. The indicator mount 1210 orients the guidance indicator 1206 at a desirable orientation and location on the proximal end 1208 of the probe 1200. The indicator mount 1210 can be a raised projection as shown that can include a planar surface. The planar surface of the indicator mount 1210 can be oriented to be parallel to an axis of the needle 1202. The planar surface can also have a normal vector that is positioned perpendicularly to the axis of the needle 1202.

The guidance indicator 1206 can include any suitable marking, crosshair, target, or other symbol that can be used to align the guidance indicator 1206 with the reference displayed by a guidance system. As shown, the guidance indicator 1206 when viewed from the top or in a plan view (FIG. 13) can appear as a circular target with a first marking and a second marking positioned perpendicularly to each other. In other examples, the guidance indicator 1206 can have other appearances and markings.

In the example shown, the indicator mount 1210 is a raised projection that projects outward from the handle portion 1204. In other examples, the indicator mount 1210 can be recessed or debossed into the handle portion 1204 such that a surface of the indicator mount 1210 can still include a planar surface as previously described. The guidance indicator 1206 can be printed, etched or otherwise fixed on the indicator mount 1210 as previously described with respect to guidance indicator 1006.

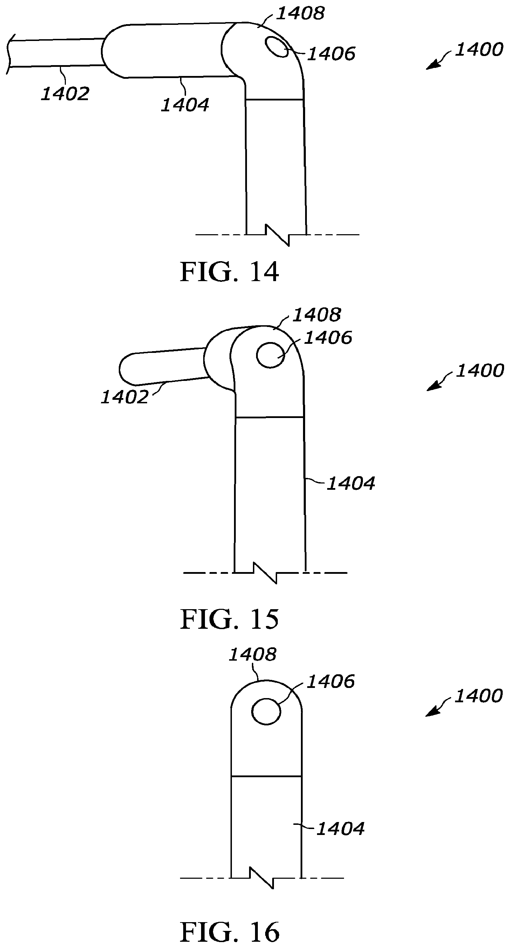

In another example, an ablation probe 1400 is shown in FIGs. 14 to 16. The ablation probe 1400 can include a needle 1402 and a handle portion 1404. The handle portion can be a molded handle, a sleeve of material or a cover for the needle 1402, tubing, wires or other internal elements of the probe 1400. The ablation probe 1400 can include a proximal end 1408 at which a guidance indicator 1406 can be positioned. The guidance indicator 1406 can be similar to the guidance indicator 1006 previously described. In this example, the guidance indicator 1406 is marked as a circular target. The guidance indicator 1406 can be used to position the proximal end 1408 of the probe 1400 in a desired location and/or orientation. The guidance indicator 1406 can be positioned collinearly with the axis of the needle 1402. In this example, the guidance indicator 1406 is positioned on the bent portion of the handle portion 1404.

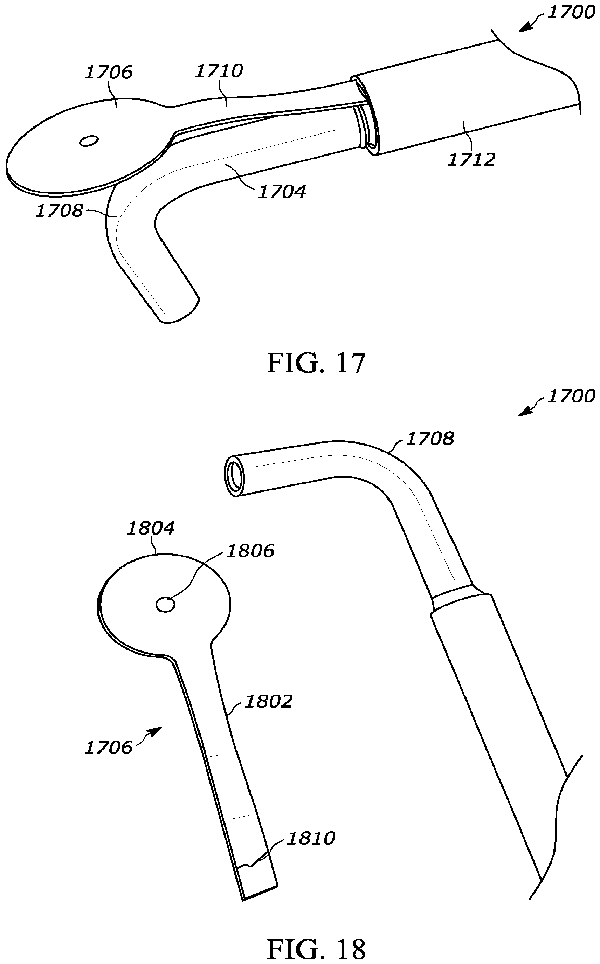

Another example ablation probe 1700 is shown in FIGs. 17 and 18. In this example, the ablation probe 1700 can include a handle portion 1704 and a proximal end 1708. In this example, the probe 1700 typically includes a needle that has been removed in the illustration shown. When included, the needle can extend from the proximal end 1708 to a distal end. The ablation probe 1700, in this example, includes a removable guidance indicator 1706. FIG. 18 shows the example guidance indicator 1706 removed from the ablation probe 1700. The guidance indicator 1706 can include a support portion 1802 and a target portion 1804. The support portion 1802 can support the target portion 1804 and connect the guidance indicator 1706 to the probe 1700. In this example, the support portion 1802 can be configured as an elongated bar of material. The support portion can have an offset 1710 that can position the target portion 1804 at a position that is raised away from the handle portion 1704. The support portion 1802 can also include connector end 1810 that can connect the guidance indicator 1706 to the probe 1700. In this example, the connector end 1810 can be received in a sleeve 1712 of the probe 1700 to hold the guidance indicator in a desired position. In other examples, the support portion 1802 can include one or more clips, collars, pins, sleeves or other connecting features that can connect the guidance indicator 1706 to the probe 1700.

The target portion 1804 can be connected to the support portion 1802 and can be configured to include a target marking 1806 or other marking that can be used to align the probe 1700 using one or more references that can be displayed by a guidance system. In this example, the target portion 1804 can have a circular shape and the target marking 1806 can be configured as a dot or other centering marking. In other examples, the target portion 1804 can have other shapes or profiles such as rectangular, diamond, oval, cross or other suitable shape. The target marking 1806 can also have other shapes or configurations and can include one or more targets, lines, symbols or other markings to align with the reference of the guidance system. The target marking 1806 can be printed, connected, stamped, etched, laser marked, embossed, debossed or otherwise fixed to the target portion 1804, including using other methods or processes previously described.

The target portion 1804 can be removably connected and positioned relative the probe 1700 such that the target portion and/or the target marking 1806 is positioned at the proximal end 1708 of the probe 1700. The target portion 1804 and/or the target marking 1806 can be further located and oriented such that the target marking 1806 is aligned with an axis of the needle (or collinearly with the needle) of the probe 1700. Guidance indicator 1706 in this example can be removed and reused during multiple ablation procedures and can be interchangeable with various ablation probes. In addition, the ablation probe 1700 can include complimentary attachment points (not shown) such as recesses, holes, pins or the like that can be used to connect and locate the guidance indicator 1706 to the probe 1700.

The guidance indicator 1706 in this example can be made of various suitable materials. In some examples, the guidance indicator 1706 can be made of a plastic material that can be molded to have a suitable shape and configuration as previously described. In other examples, the guidance indicator 1706 can be made of other materials such as metals, alloys, composites and other combinations thereof.

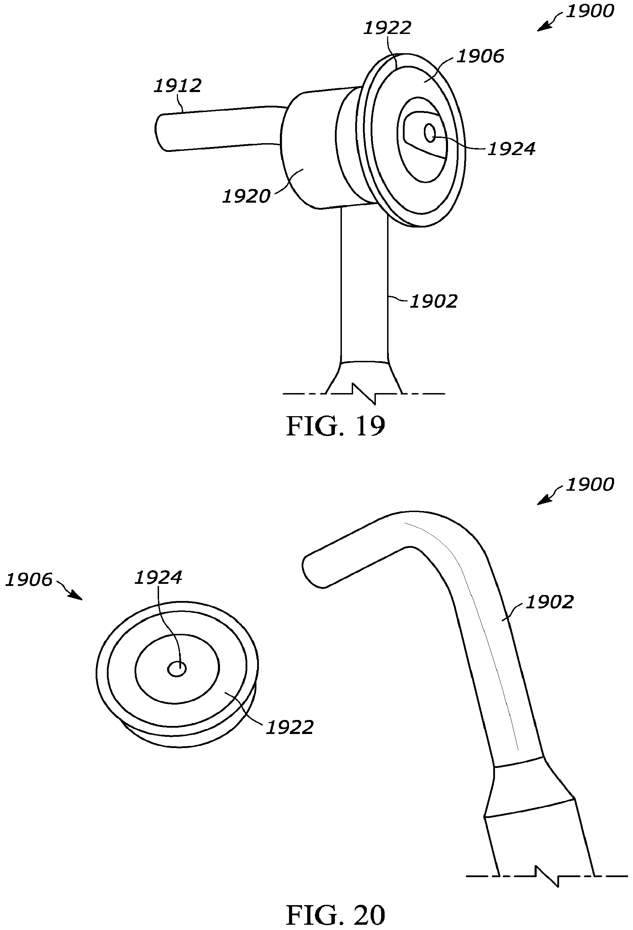

Another example ablation probe 1900 is shown in FIGs. 19 and 20. In this example shown, the probe 1900 includes a handle portion 1902 with a proximal end 1908. When the probe 1900 is in use, a needle can extend from the end 1912 shown. In this example, the probe 1900 includes a guidance indicator 1906 mounted to the proximal end 1908. The guidance indicator 1906 can be removable from the probe 1900. The guidance indicator 1906 can include a mount portion 1920 and a target portion 1922. The mount portion 1920 can be a cylindrically shaped support that can include a sleeve, groove or channel into which the handle portion 1902 (or other portion of the probe 1900) can be received to hold the guidance indicator 1906 in a desired position relative to the needle of the probe 1900. The sleeve, groove or channel or other attachment feature of the mount portion 1920 can be sized such that the guidance indicator is removable from the probe 1900 but is securely fixed to the probe 1900 during use. An interference fit or other suitable dimensional relationship between the mount portion 1920 and the probe 1900 can be used in one example. In other examples, the mount portion 1920 and/or the probe 1900 can include complimentary attachment points (not shown) such as recesses, holes, pins or the that can be used to connect and locate the guidance indicator 1906 to the probe 1900.

The guidance indicator 1906 can also include a target portion 1922. The target portion 1922 can be similarly configured as the target portion 1904 previously described. The target portion can include a target marking 1924. The target marking 1924 can be similarly configured as the target marking 1806 previously described. In the example shown, the target marking 1924 can include a centering mark that can be located collinearly with the needle of the probe 1900 at the proximal end 1908.

The guidance indicator 1906 can be made of any suitable material such as a plastic. In one example, the guidance indicator 1906 can be made of an elastomeric material that can elastically deform. Such a property of the material of the guidance indicator 1906 can allow the mount portion 1920 to deform when the tubing of the probe 1900 is inserted into the attachment channel, groove or sleeve of the mount portion 1920. In this manner, the guidance indicator 1906 can be sized and configured to fit on a variety of probes 1900.

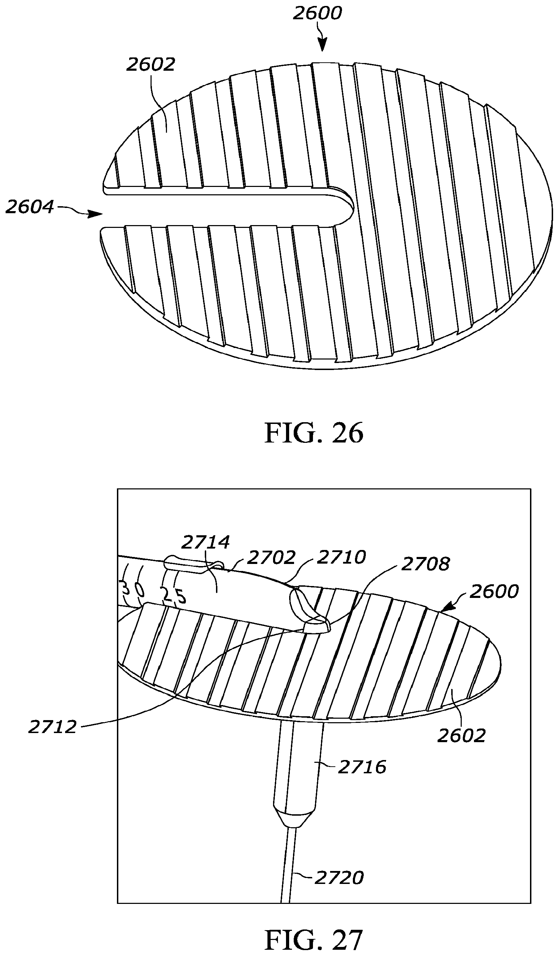

Referring now to FIGs. 26-29, another example guidance indicator 2600 is shown. The guidance indicator 2600 can be removable from a probe 2702. The guidance indicator 2600 can have an outer profile of various suitable shapes. In the example shown, the outer profile has a rounded or circular shape. The guidance indicator 2600 can be planar such that it presents a surface 2602 on which laser references of a guidance system can be projected. In this manner, the guidance indicator 2600 can allow a medical professional to easily and accurately position and align the probe 2702 in a desired location and orientation relative to a patient or subject.

As shown, the guidance indicator 2600 can also include a channel 2604. The channel 2604 can be sized to allow a probe 2702 to be positioned in the channel 2604. The channel 2604 may extend from an edge of the guidance indicator 2600 to a center of the guidance indicator 2600. When the probe 2702 is positioned in the guidance indicator 2600 (FIG. 27), the proximal end 2708 of the probe 2702 is positioned at the center of the guidance indicator 2600. The channel 2604 may have a rib or groove that can be sized to fit with a corresponding groove or rib on the probe 2702. Such a feature can locate the guidance indicator 2600 in a predetermined position relative to the probe 2702. The channel 2604 may be sized so as to have an interference fit with one or more portions of the probe 2702 to retain the guidance indicator 2600 in a desired position relative to the probe 2702. In addition, the probe 2702 and/or the guidance indicator 2600 may include a clip, tab, barb, or other retention feature to retain the guidance indicator 2600 in a desired position relative to the probe 2702.

The probe 2702 may include a first marking 2710 and/or a second marking 2712. The first marking 2710 and/or the second marking 2712 can be various suitable indicators that are marked, embossed, debossed, printed, formed, or otherwise fixed on the probe 2702 to allow references from the guidance system to be used to align the probe 2702. The first marking 2710 and the second marking 2712 can be extended from the probe 2702 and on the guidance indicator 2600. When the guidance indicator 2600 is assembled to the probe 2702, the markings of the probe can be aligned with markings on the guidance indicator 2600. The markings may be a target shape, a plus-sign shape, a cross-hair, a target symbol, or the like.

In the example shown in FIG. 27, the probe 2702 is a right-angle probe in which a first portion 2714 of the handle is at a right angle to the second portion 2716 of the handle. The second portion 2716 of the handle is aligned with the needle 2720 that may be inserted into a subject during a treatment. The guidance indicator 2600 may be aligned with the first portion 2714 of the probe 2702. In this configuration, the surface 2602 of the guidance indicator 2600 is positioned substantially perpendicularly to the needle 2720. This provides a large surface on which the references of the guidance system can be projected in order to align the needle 2720 in a desired position and orientation relative to a subject.

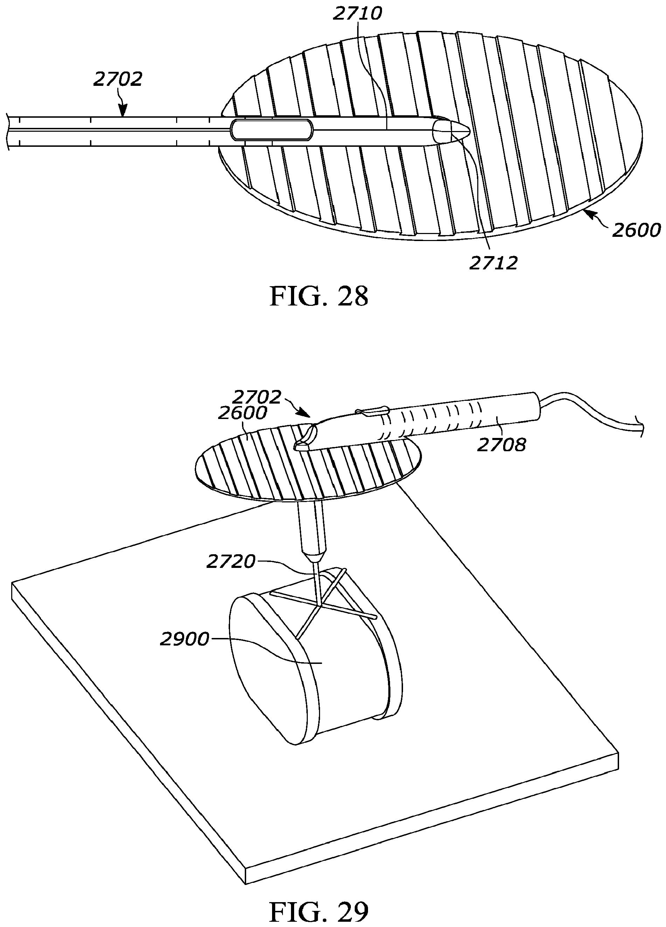

A plan view of the probe 2702 with the guidance indicator 2600 is shown in FIG. 28. As can be seen, the first marking 2710 and the second marking 2712 can be easily seen by a user in order to position the probe 2702 in a desired location and orientation. During an example procedure (FIG. 29), the distal end (i.e., the end that is first inserted into a subject during treatment) of the needle 2720 can be positioned at a location that is indicated by a guidance system. As previously described, the guidance system may, for example, project a first reference in the form of an "X", cross hairs, or other target onto the subject 2900. The distal end of the needle 2702 can be positioned at the first reference of the guidance system. The proximal end 2708 of the probe 2702 can then be aligned, with the assistance of the guidance indicator 2600, to the second reference 2502 of the guidance system. The surface of the guidance indicator 2600 allows for a user to easily view the reference from the guidance system and move the proximal end 2708 of the probe 2702 to the desired position.

The guidance indicator 2600 can be made of various suitable materials such a suitable polymer, composite or other material. The outer profile of the guidance indicator 2600 can have a rounded, circular, or elliptical shape. In other examples, the guidance indicator 2600 can have other outer profiles and shapes. The guidance indicator 2600, in this example, is configured to be used with a right angle probe but other configurations of the guidance indicator 2600 can also be used that are configured with other probes other than right angle probes as shown.

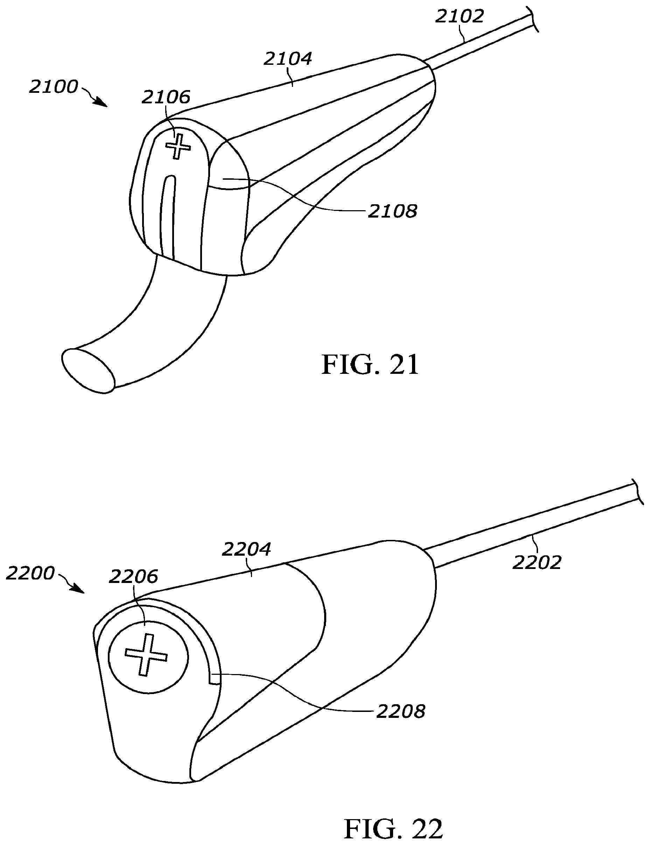

Turning now to FIGs. 21-24, more example ablation probes are shown. In many of the previous examples, the ablation probes were shown as cryoprobes that can be cooled to temperatures less than the freezing temperature of the target tissues to cause ice balls to be created that freeze and/or destroy the target tissue. The teachings and principles of the present disclosure, including the ablation probes and the guidance indicators described, can be used in various types of ablation probes including cryoprobes, RF (Radio Frequency) probes, microwave probes and other probe types. The examples shown in FIGs. 21-24 are shown as microwave ablation probes. It should be appreciated, however, that the features and descriptions are not limited to microwave ablation probes.

As shown in the FIG. 21, an example ablation probe 2100 is shown. The example probe 2100 includes a needle 2102, a handle portion 2104 and a guidance indicator 2106 located at a proximal end 2108. The guidance indicator 2106 can include any suitable marking to allow the needle 2102 to be aligned with one or more references that can be displayed by a guidance system. The guidance indicator 2106, in this example, includes a crosshair symbol that can be aligned with multiple indicators of the reference of the guidance system. The guidance indicator 2106 can be formed, printed, connected or otherwise included on the handle portion 2104. In other examples, the guidance indicator 2106 can have other shapes, symbols or markings as may be desirable.

As shown in FIG. 22, an example ablation probe 2200 is shown. The example probe 2200 includes a needle 2202, a handle portion 2204 and a guidance indicator 2206 located at a proximal end 2208. The guidance indicator 2206 can include any suitable marking to allow the needle 2202 to be aligned with one or more references that can be displayed by a guidance system. The guidance indicator 2206, in this example, includes a crosshair symbol that can be aligned with multiple indicators of the reference of the guidance system. The guidance indicator 2206 can be formed, printed, connected or otherwise included on the handle portion 2204. In other examples, the guidance indicator 2206 can have other shapes, symbols or markings as may be desirable.

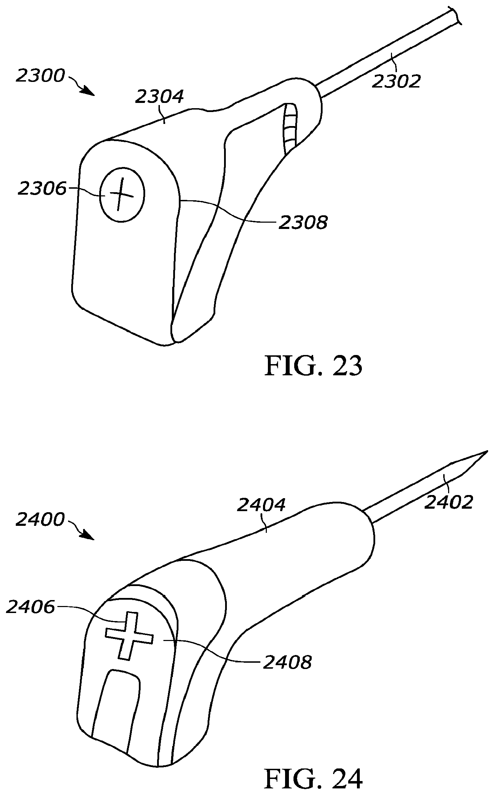

As shown in the FIG. 23, an example ablation probe 2300 is shown. The example probe 2300 includes a needle 2302, a handle portion 2304 and a guidance indicator 2306 located at a proximal end 2308. The guidance indicator 2306 can include any suitable marking to allow the needle 2302 to be aligned with one or more references that can be displayed by a guidance system. The guidance indicator 2306, in this example, includes a crosshair symbol that can be aligned with multiple indicators of the reference of the guidance system. The guidance indicator 2306 can be formed, printed, connected or otherwise included on the handle portion 2304. In other examples, the guidance indicator 2306 can have other shapes, symbols or markings as may be desirable.

As shown in FIG. 24, an example ablation probe 2400 is shown. The example probe 2400 includes a needle 2402, a handle portion 2404 and a guidance indicator 2406 located at a proximal end 2408. The guidance indicator 2406 can include any suitable marking to allow the needle 2402 to be aligned with one or more references that can be displayed by a guidance system. The guidance indicator 2406, in this example, includes a crosshair symbol that can be aligned with multiple indicators of the reference of the guidance system. The guidance indicator 2406 can be formed, printed, connected or otherwise included on the handle portion 2404. In other examples, the guidance indicator 2406 can have other shapes, symbols or markings as may be desirable.

While the guidance indicators 2106, 2206, 2306, 2406 are shown as marked on the respective handle portions, the guidance indicators can otherwise be printed, marked, stamped, embossed, debossed or inlaid on the handle or other portion of the probe for facilitation of the alignment of the guidance indicators with the reference displays of various guidance systems.

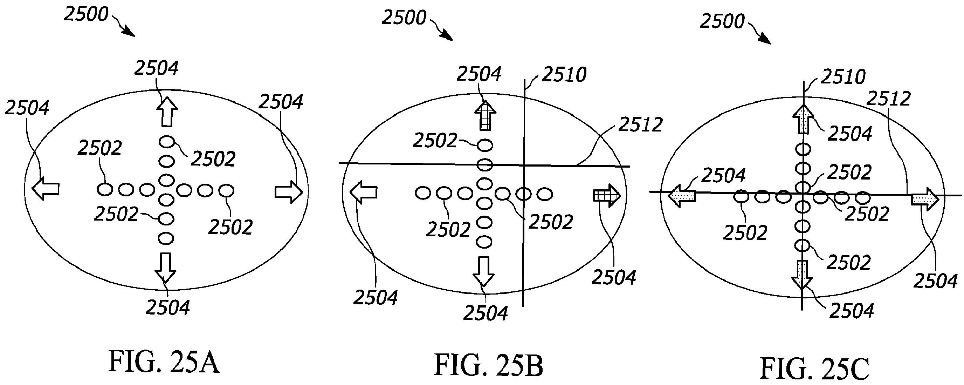

Referring now to FIGs. 25A-25C, another example guidance indicator 2500 is shown. The guidance indicator 2500 can used in any of the previously described ablation probes or incorporated into and in combination with any of the features of the guidance indicators previously described. The guidance indicator 2500 can be positioned on a permanently integrated guidance indicator or be incorporated into a removable guidance indicator such as guidance indicators 1706, 1906. The guidance indicator 2500 can be positioned at a proximal end of an ablation probe and can be aligned with an axis of the needle of the probe or be collinearly aligned with the needle of the probe.

As shown, the guidance indicator 2500 can include an array of photo sensors 2502. The photo sensors 2502 can be configured in an array or other pattern that can be coordinated with a pattern or shape of the reference that can be displayed by the guidance system. In the example shown, the array of photo sensors is arranged in a cross shape to coordinate with the crosshair pattern of the reference displayed by the guidance system, such as the second reference 302 (FIG. 3) previously described. In other examples, the array of photo sensors can have other shapes or patterns such as a bullseye and target pattern, one or more concentric circles, an x-pattern or the like.

The photo sensors 2502 can be any suitable sensor that can detect an alignment of the reference of the guidance system. For example, the photo sensors 2502 can be photo cells or other optical sensors that can detect an alignment of a laser light of the second reference 302 (FIG. 3) of the guidance system 120. In other examples, other suitable sensors can be used.

The guidance indicator 2500 can also include one or more alignment notifications 2504. The alignment notifications 2504 can be any suitable communicator that can provide a notification to the user that the guidance indicator 2500 is aligned with the array of photo sensors 2502. In this example, the alignment notifications 2504 are arrow-shaped shapes that can illuminate when the reference from the guidance system is aligned with the array of photo sensors 2502. For example, the alignment notifications 2504 can illuminate and/or change color when the photo sensors 2502 located adjacent to the alignment notifications 2504 are aligned with the reference display from the guidance system. In other examples, the alignment notifications can be other shapes, or provide other or additional signals to the user of alignment such as audible notifications, flashing notifications, tactile notifications (vibrations) or the like.

During use, the guidance indicator 2500 can operate in a few different states. In a misaligned state (as shown in FIG. 25B), the reference cross hair of the guidance system that can include a first marker 2510 and a second marker 2512 is not aligned with the array of photo sensors 2502. The alignment notifications 2504 can remain unilluminated when such misalignment is present. In some examples, the alignment notifications 2504 can indicate a direction and/or an indication of the misalignment. In the example shown, the crosshair is misaligned in a first quadrant of the array of photo sensors 2502. In this instance, the top and right-side alignment notifications 2504 can illuminate to indicate the misalignment of the crosshair and the array of photo sensors. Similarly, if the crosshair were misaligned in the third quadrant of the array of photo sensors 2502 (i.e. bottom-left quadrant), the bottom and the left-side alignment notifications 2504 can illuminate to indicate the location of the misalignment. In other examples, other notifications can be provided to indicate the misalignment and/or the direction that the user needs to move the ablation probe to achieve alignment.

As shown in FIG. 25C, the guidance indicator 2500 can also operate in an aligned state. In the aligned state, the crosshair can be aligned with the array of photo sensors 2502. When such alignment is achieved, one or more of the alignment notifications 2504 can illuminate or otherwise emit a signal to notify the user of alignment. In the example shown, when alignment is achieved, all four alignment notifications 2504 can illuminate.

As can be appreciated, the guidance indicator 2500 can include other sensors, other alignment notifications and other user interfaces such as LCD screens or the like to communicate with the user to notify the user of alignment and of misalignment as well as providing feedback to the user regarding alignment of the probe to the reference display and/or the predetermined probe location and/or the probe orientation.

Various methods are also contemplated by the present disclosure. In some embodiments, methods of positioning an ablation probe using a guidance system and/or a guidance indicator are contemplated. In one method, a guidance system can be used to display one or more references on a subject. The references can be laser references that project a crosshair or other symbol on the subject.

A guidance indicator can be coupled to an ablation probe. Any suitable guidance indicator can be used. Any of the example guidance indicators can be used in this step of the method. The guidance indicator can be integrally formed with the ablation probe (i.e., not removable) or in the handle portion of the ablation probe. In other examples, the guidance indicator can be removable and this step contemplates attaching the guidance indicator to the ablation probe. The guidance indicator can be attached to a proximal end of the ablation probe and can be aligned with a needle of the ablation probe as previously described.

The method can continue by aligning a distal end of the ablation probe to a first reference from the guidance system. The first reference can indicate an entrance point for the ablation probe into the subject. The user can move the ablation probe and position the distal end of the probe at or aligned with the first reference.

The method can then include aligning a proximal end of the ablation probe with a second reference from the guidance system. The second reference can generally indicate an orientation of the probe relative the first reference. The guidance indicator can be used to align the proximal end of the probe with the second reference. The guidance indicators of the present disclosure can make this step easier than existing probes because the guidance indicators can include markings, sensors or other alignment features with the second reference.

When the ablation probe is in an aligned position, the user (i.e. the medical professional) can perform the treatment procedure on the subject in a manner that will allow the ablation probe to be guided and/or positioned in a desired manner with respect to the target tissue. In this manner, the likelihood that the ablation probe will be positioned in a desirable position relative to the target tissue and with minimal or reduced damage to surrounding health tissue and body structures is improved over existing probes and methods.

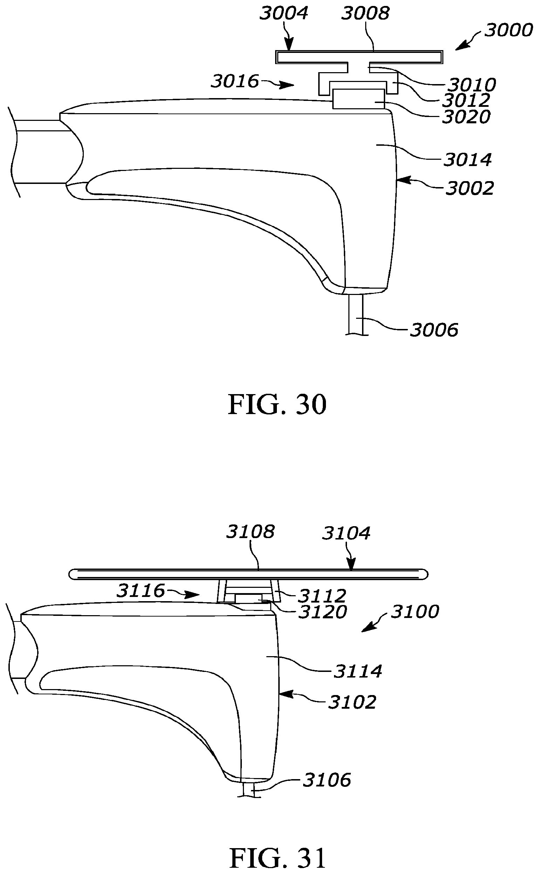

Referring now to FIG. 30, another example ablation probe assembly 3000 is shown. In this example, the ablation probe assembly 3000 includes an ablation probe 3002 and a guidance indicator 3004. The guidance indicator 3004 may be removably attached to the ablation probe 3002 to allow a position and/or orientation of the needle 3006 to be determined using a laser guidance apparatus as previously described. The guidance indicator 3004 may include an indicator platform 3008 with an indicator surface that faces upwardly during use to allow a first reference and/or a second reference to be used during an ablation procedure. The first reference and/or the second reference (see FIG. 5, for example) may be projected by the laser guidance system and the indicator platform provides a large surface for the user to align the probe 3002.

The indicator platform 3008 may be shaped to have a suitable size to allow visualization of the indicators of the laser guidance system. In some examples, the indicator platform 3008 may be circular in shape. In other examples, the indicator platform may have other shapes such as oval, octagon, U-shape, or other shapes. In some examples, a width or diameter of the indicator platform (when viewed in the plan view) may be larger than a width of a handle 3014 of the ablation probe 3002. In some examples, the width or diameter of the indicator platform may be at least two times larger than a width of the handle 3014. In still other examples, the width or diameter of the indicator platform 3008 may be at least 3x, 4x, or 5x greater than a width of the handle 3014.

The guidance indicator 3004 may also include a mounting portion 3016 that can be attached or coupled to the indicator platform 3008. The mounting portion 3016 can couple the guidance indicator 3004 to the ablation probe 3002. In the example shown in FIG. 30, the mounting portion 3016 may include an extension 3010 and a retention cup 3012. The extensions 3010 may be connected to the indicator platform 3008 and extend away from the indicator platform 3008 on a side opposite to the indicator surface. The retention cup 3012 may be connected to the extension 3010. The retention cup 3012 may define a cavity or space in which a complimentary structure of the ablation probe 3002 can be received to couple the guidance indicator 3004 to the ablation probe 3002 in a predetermined position. In some examples, the retention cup 3012 may be cylindrical. In other examples, the retention cup may have other shapes.

The ablation probe 3002 may include an attachment post 3020. The attachment post 3020 may be sized complimentarily to the cavity of the retention cup 3012. Thus, the attachment post 3020 can be received into the retention cup 3012 to retain and support the guidance indicator 3004 in the predetermined position. As can be appreciated, the attachment post 3020 can be similarly shaped or complimentarily shaped relative to the retention cup 3012 so that the post 3020 can fit in the retention cup 3012. The post 3020 can be cylindrically shaped with a diameter sized slightly less than the inner diameter of the retention cup 3012, for example. In other examples, the post 3020 and the retention cup 3012 can sized so that an interference fit exists between the mating surfaces.

The retention cup 3012 and/or the post 3020 may include a locking feature or a coupling feature that can releasably hold or maintain the guidance indicator 3004 in the installed position on the post 3020. It may be desirable, however, to allow the guidance indicator 3004 to be easily removed from the ablation probe 3002. In some instances, a user may desire to remove the guidance indicator 3004 from the ablation probe 3002 after the probe 3002 has been positioned and verified. In other instances, it is desirable that the guidance indicator 3004 disengages if a user bumps, contacts, or otherwise exerts a force on the guidance indicator 3004 during a procedure. It is desirable under such circumstances that the guidance indicator 3004 is disengaged from the probe 3002 rather than staying fixed and such contact moving the needle 3006 during a procedure.

The locking feature or coupling feature may be any suitable connection to allow the guidance indicator 3004 to be disengaged at a predetermined force level. In some examples, the retention cup 3012 and/or the post 3020 may include a magnet. In some examples, both the retention cup 3012 and the post 3020 include a magnet that are oriented with opposite polarities so that the magnetic forces of the magnets urge the post 3020 to be received into the retention cup 3012. In other examples, the retention cup 3012 includes a magnet and the post 3020 includes a metallic (i.e., a ferrous metal disc, other ferrous element, or other magnetically attracted element). In still other examples, the post 3020 may include a magnet and the retention cup 3012 may include a ferrous metal disc or other ferrous element. In these examples, the magnet urges the retention cup 3012 and the post 3020 into an engaged position with the post 3020 received into the retention cup 3012. A user can disengage the retention cup 3012 from the post 3020 by overcoming the magnetic force. Similarly, if the guidance indicator 3004 is contacted or bumped during a procedure, the guidance indicator 3004 can become disengaged from the ablation probe 3002 rather than the contact or bump moving the needle 3006 of the ablation probe 3002.

In other examples, the locking feature or coupling feature may include other elements other than or in addition to the magnet. A suitable interference fit may be used, for example. In other examples, the retention cup 3012 may include a rib, dimple, protrusion, or other feature that can be used to retain the guidance indicator 3004 to the ablation probe 3002. In other examples, other coupling features may be used.

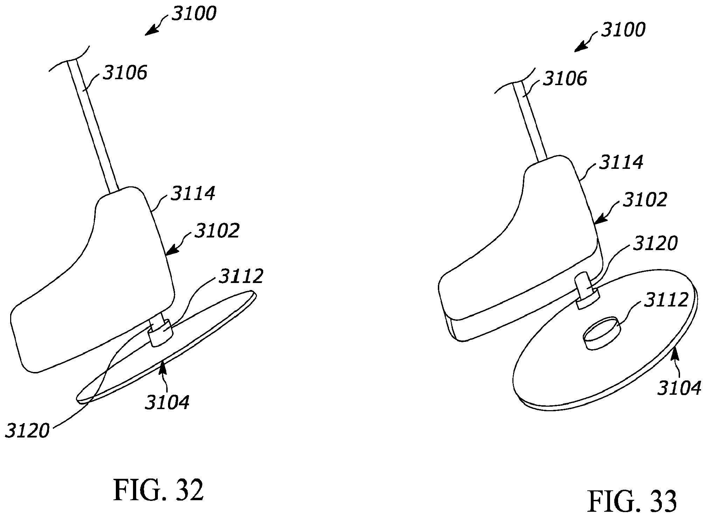

Turning now to FIGs. 31-33, another example ablation probe assembly 3100 is shown. The ablation probe assembly 3100 is similar to the ablation probe assembly 3000 previously described. The ablation probe assembly 3100 includes a guidance indicator 3104 and ablation probe 3102. The guidance indicator 3104 may be removably attached to the ablation probe 3102 to allow a user to align the needle 3106 of the ablation probe 3002 to insert and/or install the needle 3106 proximate to a target tissue using a laser guidance system as previously described.

The guidance indicator 3104 may include an indicator platform 3108 that includes a retention cup 3112. The retention cup 3112 may be positioned on a side of the indicator platform 3108 opposite to the indicator surface. The retention cup 3112 may be sized and shaped to receive a post 3120 on the handle 3114 of the ablation probe 3102. The retention cup 3112, in this example, may include a locking or coupling feature such as a magnet to secure the guidance indicator 3104 to the ablation probe 3102 while allowing easy removal or disengagement for the reasons noted above.

The guidance indicator 3104 may be formed of a suitable plastic or other material. In some example, the retention cup 3112 and the guidance indicator 3104 can be integrally formed during an injection molding process or other process such as 3D-printing or additive manufacturing. The magnet or other coupling feature can be inserted into the guidance indicator 3004 during the same process, in some examples.

The attachment post 3120 can also be formed of a suitable plastic, polymer, composite or other suitable material. The magnet may integrally formed into the post 3120 or can be fixed to the post using a suitable attachment process such as adhesive or the like.

In other examples, the post 3120 and/or the retention cup 3112 can be formed of an elastic material such as a suitable polymer or rubber material. The post 3120 can be made of an elastic material such that the post 3120 can deform or flex but return to an original position. Such flexibility of the post 3120 and/or the retention cup 3112 can allow the guidance indicator to be contacted or bumped during a procedure without moving the needle 3106 of the ablation probe.

As can be appreciated and as previously described, the position of the guidance indicator 3104 on the ablation probe 3102 can allow the needle 3106 to be positioned, oriented and inserted proximate to a target tissue in a patient. The guidance indicator 3104 may be positioned to be centered relative the needle 3106. Thus, the retention cup 3112 may be centered relative to an axis of the needle 3106 such that a center of the retention cup is aligned with the axis of the needle 3106. A center of the post 3120 may also be aligned with the axis of the needle 3106. The indicator surface of the indicator platform 3108 may be positioned substantially perpendicularly to the axis of the needle 3106 so that the surface can be used to view and align one or more markings on the indicator surface with the references of the laser guidance system. The indicator surface may include a crosshair, plus sign, X, bullseye, circle, or other markings that may be used for this purpose.

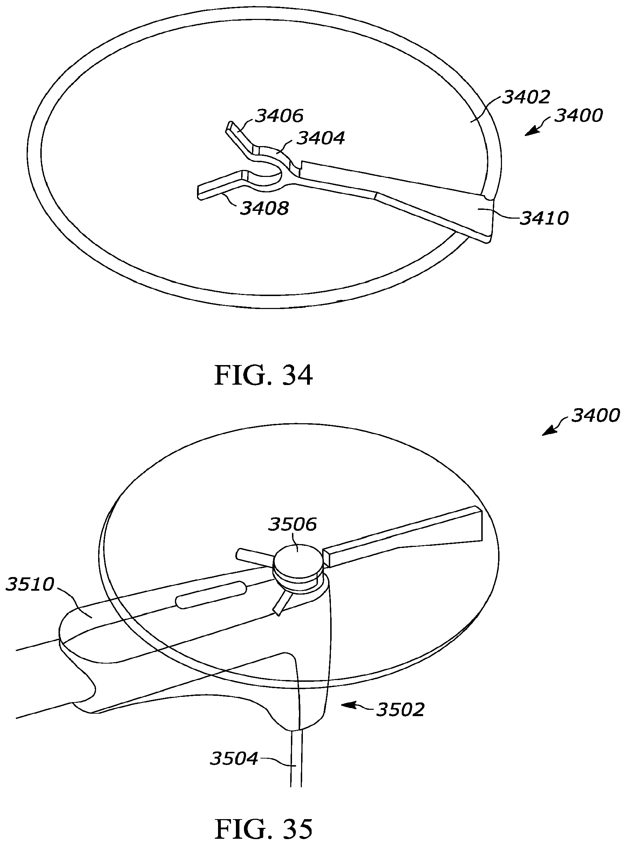

Another example guidance indicator is shown in FIGs. 34-36. In this example, the guidance indicator 3400 may be used in a similar manner to that described above. In this example, the guidance indicator 3400 may include an indicator platform 3402, a coupling ring 3404, one or more guide ribs 3406, 3408, and a handle 3410. The coupling ring 3404, the one or more guide ribs 3406, 3408, and the handle 3410 may be positioned on a side of the indicator platform 3402 opposite to the indicator surface.

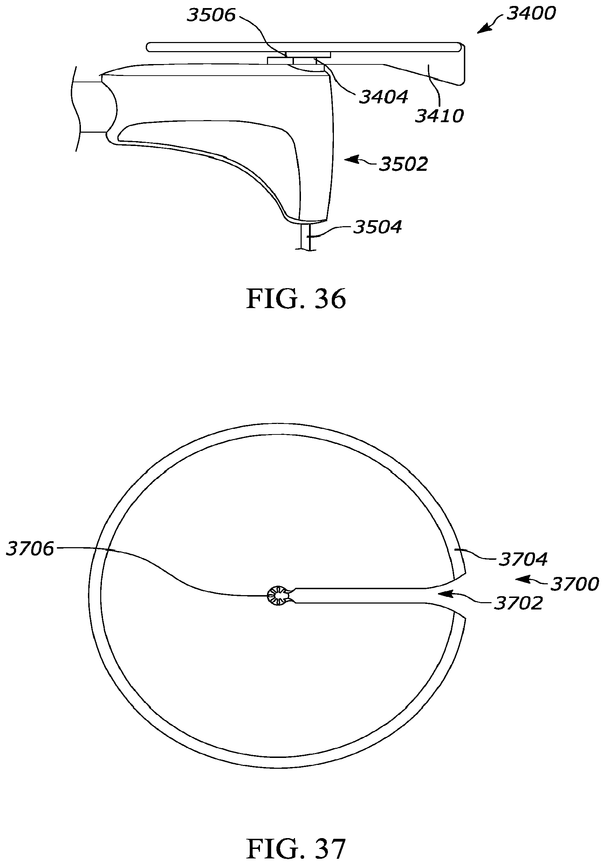

The guidance indicator 3400 may be coupled to an ablation probe 3502 on a side of a handle 3510 opposite to the needle 3504. The guidance indicator 3400 may be coupled to a post 3506 that may extend upwardly from the handle 3510 opposite to the needle 3504. As shown in FIG. 36, the handle 3410 may support the coupling ring 3404 at a position so that the coupling ring 3404 is vertically spaced below a bottom surface of the indicator platform 3402. The post 3506 may include a disc or head that has an outer diameter that is larger than an internal diameter of the coupling ring 3404. The post 3506 can be received in the coupling ring 3404 so that the disc or head of the post 3506 is positioned above the coupling ring 3404 and the shaft of the post 3506 is positioned in the coupling ring 3404.

The guide ribs 3406, 3408 may be connected to the coupling ring 3404 and angled outwardly relative to a center of the coupling ring 3404. The guide ribs 3406, 3408 can guide the post 3506 into the coupling ring 3404. When the post passes the arcuate opening in the coupling ring 3404, the post can be retained in the coupling ring 3404 with the disc or head of the post 3506 positioned between the bottom surface of the indicator platform 3402 and the upper surface of the coupling ring 3404. The shaft of the post 3506 being located concentrically within the coupling ring 3404. In this position, the guidance indicator is retained in position relative to the ablation probe 3502. A center of the coupling ring 3404 can be aligned with an axis of the needle 3504 and the axis of the post 3506 so that the guidance indicator can be used to align the position and orientation of the needle 3504 during an ablation procedure.

The handle 3410 may be positioned on the indicator platform 3402 to support the coupling ring 3404. In addition, the handle 3410 may be used by an operator to grip the guidance indicator 3400 during installation or removal from the from the ablation probe 3502. The size and flexibility of coupling ring 3404 can be configured so that the installation and removal of the guidance indicator is less or equal to a predetermined installation force. In this manner, the guidance indicator 3400 can be easily installed and removed during ablation procedures.

Referring now to FIG. 37, another example guidance indicator 3700 is shown. In this example, the guidance indicator 3700 may include an indicator platform 3704 that defines a channel 3702. The channel 3702 may extend from an edge of the indicator platform 3704 to a center 3706. The channel 3702 may allow a user to install the guidance indicator 3700 onto a needle of an ablation probe (not shown). As can be appreciated, the center 3706 may include one or more fingers that extend inwardly in the center 3706. The channel 3702 may be sized to have a width larger than an outer diameter of an ablation needle. The guidance indicator 3700 can be guided over the needle until the needle is seated in the center 3706. The fingers in the center 3706 may be deformable and may extend inwardly so that the distance between the fingers in the center 3706 is less than the outer diameter of the needle. The fingers may deform and exert a force against the needle when the needle is positioned in the center 3706 to retain the needle at the center 3706 of the guidance indicator.

The guidance indicator 3700 may include other features such as markings on the indicator platform 3704 to allow a user to align the needle of the ablation probe using a laser guidance system as previously described.

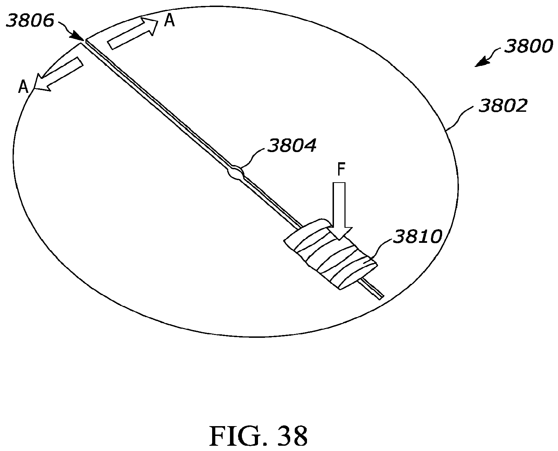

Referring now to FIG. 38, another example guidance indicator 3800 is shown. The guidance indicator 3800 may include an indicator platform 3802, a channel 3806, a center 3804, and one or more actuators 3810. The guidance indicator 3800 may be configured to allow a needle of an ablation probe (not shown) to be installed into the indicator platform 3802 so that the needle is positioned at the center 3804. The guidance indicator can then be used to align the needle with the use of a laser guidance system as previously described.

In this example, in first state (or relaxed state) the channel 3806 may be sized so that the width of the channel 3806 is smaller than an outer diameter of the needle. As such, the guidance indicator 3800 cannot be installed onto the ablation probe in the first state. The guidance indicator 3800 can be moved from the first (or relaxed) state to an installation state in which a width of the channel 3806 increases by movement of the indicator platform at the edge of the channel in the direction indicated by the arrows marked A. When the channel 3806 widens the guidance indicator 3800 can be slid along the needle until the needle reaches the center 3804. The guidance indicator can then be returned to the first (or relaxed) state to retain the guidance indicator 3800 on the needle with the needle positioned at the center 3804.

The guidance indicator 3800 may include one or more actuators 3810. The actuators 3810 can be used to move the guidance indicator 3800 from the first (or relaxed) state to the installation state. The actuators 3810, in this example, are arced bands of material that span across an opening at opposite sides of the channel 3806. The actuators 3810 may bend upwardly away from the surface of the indicator platform 3802. When a downward force (as indicated by the arrow F) is exerted on the actuators, the actuators straighten from their arced shape and push the opposite sides of the channel 3806 outwardly. In this manner, a force F exerted on the actuators 3810 can open or widen the channel 3806 in the direction indicated by the arrows marked A and move the guidance indicator from the first (or relaxed) state to the installation state. When the force F is released from the actuators 3810, the channel 3806 can return to its original (relaxed) state.

In other examples, the guidance indicator 3800 may have other actuator that can move the indicator platform from the first state to the installation state. Such other actuators or features may include a pinch feature, handles, ribs, holes or other formations that can be used by a user to widen the channel 3806 during installation of the guidance indicator 3800.

The example methods and apparatuses described herein may be at least partially embodied in the form of computer-implemented processes and apparatus for practicing those processes and/or the described functionality. The disclosed methods may also be at least partially embodied in the form of tangible, non-transient machine readable storage media encoded with computer program code. The media may include, for example, RAMs, ROMs, CD-ROMs, DVD-ROMs, BD-ROMs, hard disk drives, flash memories, or any other non-transient machine-readable storage medium, or any combination of these mediums, wherein, when the computer program code is loaded into and executed by a computer, the computer becomes an apparatus for practicing the method. The methods may also be at least partially embodied in the form of a computer into which computer program code is loaded and/or executed, such that, the computer becomes an apparatus for practicing the methods. When implemented on a general-purpose processor, the computer program code segments configure the processor to create specific logic circuits. The methods may alternatively be at least partially embodied in a digital signal processor formed of application specific integrated circuits for performing the methods.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A guidance indicator (3004, 3104, 3400) comprising:
an indicator platform (3008, 3108, 3402) having an indicator surface; and
a mounting portion (3016) connected to the indicator platform and supporting the indicator surface in a predetermined orientation relative to a needle (3006, 3106, 3504) of an ablation probe (3002, 3102, 3502), the mounting portion adapted to removably attach to a complimentary attachment post (3020, 3120, 3506) of the ablation probe.

2. The guidance indicator of claim 1, wherein the mounting portion comprises a retention cup (3012, 3112) that includes a magnet to removably couple the guidance indicator to the attachment post.

3. The guidance indicator of claim 2, wherein the magnet is configured to removably couple to a complimentary magnet in the attachment post.

4. The guidance indicator of claim 1, wherein the mounting portion comprises one or more guide ribs (3406, 3408) positioned proximate a coupling ring (3404), the guide ribs angled toward a center of the coupling ring to guide the attachment post into the coupling ring during assembly of the guidance indicator to the ablation probe.

5. The guidance indicator of claim 4, further comprising a handle (3410) connected to the indicator platform (3402) on a side opposite to the indicator surface.

6. The guidance indicator of claim 1, wherein the mounting portion is configured to removably attach to the attachment post via an interference fit.

7. The guidance indicator of any one of claims 1 to 6, wherein the mounting portion is connected at a center of the indicator platform opposite to the indicator surface.

8. The guidance indicator of any one of claims 1 to 7, wherein the indicator surface comprises a first marking and a second marking, the second marking oriented perpendicular to the first marking, the first marking and the second marking configured to align to one or more lasers of a laser guidance system.

9. The guidance indicator of any one of claims 1 to 8, wherein the predetermined orientation comprises an orientation of the indicator surface that is substantially perpendicular to an axis of the needle.

10. The guidance indicator of any one of claims 1 to 9, wherein the mounting portion comprises a retention cup and a center of the retention cup is aligned with an axis of the needle when positioned on the ablation probe.

11. An ablation probe (3002, 3102, 3502) comprising the guidance indicator of any one of claims 1 to 10.

12. The ablation probe of claim 11, wherein the attachment post comprises an elastic material.

13. The ablation probe of claim 11 or claim 12, wherein the attachment post is flexible and biased to maintain the indicator surface in the predetermined orientation.

14. The ablation probe of any one of claims 11 to 13, comprising an ablation probe handle (3014, 3114), the needle extending away from the ablation probe handle on a first side and the attachment post extending away from the ablation probe handle on a second side opposite to the first side.

15. The ablation probe of claim 14, wherein an overall width of the indicator surface is greater than a width of the second side of the ablation probe handle and optionally the overall width of the indicator surface is at least twice the width of the second side of the ablation probe handle.
